(19) **Europäisches Patentamt / European Patent Office / Office européen des brevets**

(11) **EP 3 216 392 A1**

(12) **EUROPEAN PATENT APPLICATION**
published in accordance with Art. 153(4) EPC

(43) Date of publication:
**13.09.2017 Bulletin 2017/37**

(51) Int Cl.:
*A61B 5/0476* (2006.01)  *A61B 10/00* (2006.01)

(21) Application number: **15867759.1**

(22) Date of filing: **24.11.2015**

(86) International application number:
**PCT/JP2015/082844**

(87) International publication number:
**WO 2016/093048 (16.06.2016 Gazette 2016/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.12.2014 JP 2014248953**

(71) Applicant: **NTT Data I Corporation**
**Shinjuku-ku, Tokyo 162-0824 (JP)**

(72) Inventors:
- **MOMOSE, Kimihisa**
  **Tokyo 162-0824 (JP)**
- **KAKIMOTO, Kazunori**
  **Tokyo 162-0824 (JP)**
- **MATSUNAGA, Hiroshi**
  **Tokyo 162-0824 (JP)**
- **OKUYAMA, Takashi**
  **Tokyo 162-0824 (JP)**
- **TSUTSUMIDA, Toshio**
  **Tokyo 162-0824 (JP)**

(74) Representative: **Brevalex**
**95, rue d'Amsterdam**
**75378 Paris Cedex 8 (FR)**

(54) **BRAIN DISEASE DIAGNOSIS ASSISTANCE SYSTEM, BRAIN DISEASE DIAGNOSIS ASSISTANCE METHOD, AND PROGRAM**

(57) A brain disease diagnosis assistance system acquires a plurality of learning data items, each including brain wave feature data including feature amounts of brain waves extracted from the brain waves and disease information attached to the brain wave feature data, the disease information indicating a state of a brain disease corresponding to the brain wave feature data and classifies the plurality of acquired learning data into a plurality of clusters. A classifier for classifying learning data into portions corresponding respectively to types of disease information is generated based on disease information attached to learning data in each classified cluster. Then, brain wave feature data of a subject is acquired and a cluster to which the brain wave feature data is classified is specified and one of a plurality of brain diseases, which corresponds to the brain wave feature data of the subject, is determined through the generated classifier.

FIG. 1

**Description**

[Technical Field]

The present invention relates to a brain disease diagnosis assistance system, a brain disease diagnosis assistance method, and a program.

Priority is claimed on Japanese Patent Application No. 2014-248953, filed December 9, 2014, the content of which is incorporated herein by reference.

[Background Art]

With the arrival of an aging society, there has arisen a need to take measures against dementia as a brain disease. The following description will be given with reference to dementia as an example. The progression of dementia is delayed through medication and dementia can sometimes be improved by operation. Early recognition of dementia is important to take effective measures against such dementia.

For example, a device which measures brain activity using the fact that degradation of neuronal functions in the brain cortex makes neuronal activity unstable and this influence results in local fluctuations of the powers of brain waves over an entire frequency band of brain waves is described in Patent Literature 1. In this technology, averages or standard deviations of values (hereinafter referred to as brain wave feature data), to which brain waves detected by sensors attached to the head of a subject at a plurality of positions thereon are standardized in each predetermined frequency band, are obtained and a Z score of the averages of the subject is obtained by comparing the averages with averages of brain wave feature data which have been obtained for a group of normal persons in the same manner. A degraded part of the brain functions of the subject can be indicated based on the Z score of the subject.

In Patent Literature 1, it is also described that a Z score of a group of patients with Alzheimer's dementia is calculated using the same method and the calculated Z score is used as a template indicating the characteristics of the disease and then a coefficient of correlation between a Z score of each individual subject and the template is obtained, whereby the similarity therebetween can be numerically displayed.

[Citation List]

[Patent Literature]

[Patent Literature 1]
Japanese Patent No. 4145344

[Summary of Invention]

[Technical Problem]

However, the method of Patent Literature 1 has the following problems. For example, in the method of Patent Literature 1, the similarity between brain wave feature data of a subject and brain wave feature data of a group of normal persons or brain wave feature data of a group of patients with Alzheimer's dementia is determined using the Z score. When the determination is made using the Z score, it is assumed that the statistical population follows a normal distribution. However, there is no guarantee that the brain wave feature data of the group of normal persons or the brain wave feature data of the group of patients with Alzheimer's dementia follows a normal distribution. Accordingly, to use the Z score, there may be a need to extract appropriate data through trial and error such that data of the statistical population follows a normal distribution. In addition, in the international standard, brain waves are measured at 19 positions and, for example, in the method of Patent Literature 1, brain waves are measured at the 19 positions and 2 additional positions, i.e., at a total of 21 positions. In any case, brain wave feature data is multidimensional and it is difficult to extract suitable data, which follows a normal distribution, from a plurality of multidimensional data.

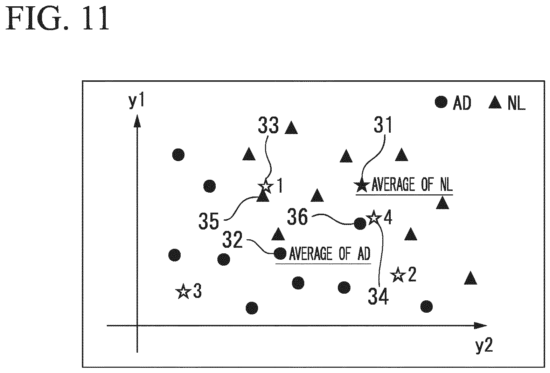

In the method of Patent Literature 1, the similarity between brain wave feature data of a subject and brain wave feature data of a group of normal persons or a group of patients with Alzheimer's dementia is determined based on the distance between average values of the brain wave feature data of the subject and the group. However, the average value of the brain wave feature data of the group of normal persons does not necessarily converge to a value indicating the characteristics of brain wave feature data of normal persons. Similarly, the average value of the brain wave feature data of the group of patients with Alzheimer's dementia does not necessarily converge to a value indicating the characteristics of brain wave feature data of patients with Alzheimer's dementia. This is described as follows with reference to Fig. 11. The description will be given with reference to an example in which two pieces of brain wave feature data are used for ease of explanation. A vertical axis (y1 axis) and a horizontal axis (y2 axis) represent the two pieces of brain wave feature data. Thus, brain wave feature data of a person can be represented by a point having coordinates corresponding to values of the two pieces of brain wave feature data. A point shown as a triangle indicates brain wave feature data of a normal person (NL). A point shown as a circle indicates brain wave feature data of a patient diagnosed with Alzheimer's dementia (AD). A point shown as a star indicates brain wave feature data of a subject. Here, the average value of NL is represented by a star point 31. The average value of AD is represented by a circle point 32.

Here, whether the brain wave feature data of the subject is classified as that of a normal person or that of a patient with Alzheimer's dementia can be considered based on the average values of the respective brain wave feature data of normal persons and patients with Alzheimer's dementia. A method in which an average value of NL and an average value of AD are assumed as representative values of NL and AD and the distances between brain wave feature data of a subject and the representative values are compared and the brain wave feature data of the subject is classified as one of NL and AD with the smaller distance can be considered as an example of a method of classifying brain wave feature data of a subject as one of NL and AD. For example, brain wave feature data of subject 1 (star point 33) is near the average value of AD (circle point 32). Brain wave feature data of subject 4 (star point 34) is near the average value of NL (star point 31). However, a single piece of brain wave feature data of NL (triangular point 35) is present near the brain wave feature data of subject 1 (star point 33). A single piece of brain wave feature data of AD (circle point 36) is also present near the brain wave feature data of subject 4 (star point 34). According to the method of Patent Literature 1, in this case, there is also a possibility that subject 1 (star point 33) is determined to be AD and subject 4 (star point 34) is determined to be NL. However, the average value of brain wave feature data is not necessarily a value correctly indicating the characteristics of brain wave feature data of a symptom or a state of activity. Moreover, brain wave feature data is multidimensional as described above and it is difficult to appropriately converge to the representative value of such multidimensional data through average calculation.

The present invention provides a brain disease diagnosis assistance system, a brain disease diagnosis assistance method, and a program which can solve the problems described above.

[Solution to Problem]

According to a first aspect of the present invention, a brain disease diagnosis assistance system includes an evaluation model calculation unit which acquires a plurality of learning data items, each including brain wave feature data including feature amounts of brain waves extracted from the brain waves and disease information attached to the brain wave feature data, the disease information indicating a state of a brain disease corresponding to the brain wave feature data, and calculates an evaluation model that performs brain disease determination through machine learning with the plurality of learning data items, and a determination unit which acquires brain wave feature data of a subject and performs determination of a brain disease indicated by the brain wave feature data of the subject on the basis of the evaluation model.

According to a second aspect of the present invention, the brain disease diagnosis assistance system further includes an input unit which receives an input of the disease information to be attached to the brain wave feature data of the subject, wherein the input unit allows a new learning data item including the brain wave feature data associated with the received disease information to be stored in a storage unit, and the evaluation model calculation unit adds the new learning data item to the plurality of learning data items to perform calculation of an evaluation model.

According to a third aspect of the present invention, a plurality of the feature amounts are included in the brain wave feature data, and, for brain wave feature data included in each of the plurality of learning data items, the evaluation model calculation unit extracts one or a plurality of feature amounts effective for calculating an evaluation model and calculates the evaluation model using only the extracted feature amounts.

According to a fourth aspect of the present invention, information indicating one or a plurality of types of brain disease is included in disease information of the plurality of learning data items, the evaluation model calculation unit calculates boundary information that separates the plurality of learning data items into portions corresponding respectively to types of disease information attached to the learning data items, and the determination unit performs determination of a brain disease indicated by the brain wave feature data of the subject on the basis of the boundary information.

According to a fifth aspect of the present invention, the evaluation model calculation unit classifies the plurality of brain wave feature data into a plurality of groups by clustering based on the feature amounts, and the determination unit determines a group to which the brain wave feature data of the subject is classified from among the groups into which the evaluation model calculation unit has classified the plurality of brain wave feature data.

According to a sixth aspect of the present invention, information indicating one or a plurality of brain diseases is included in disease information of the plurality of learning data items, the evaluation model calculation unit calculates, for each type of disease information, a proportion of learning data to which the type of the disease information is attached in learning data included in the group to which the brain wave feature data of the subject has been determined to be classified by the determination unit, and the determination unit performs determination of a brain disease indicated by the brain wave feature data of the subject on the basis of the proportion.

According to a seventh aspect of the present invention, the evaluation model calculation unit calculates boundary information that separates the learning data included in the group to which the brain wave feature data of the subject has been determined to be classified by the determination unit into portions corresponding respectively to types of disease information attached to the learning data items, and the determination unit performs determination of a brain disease indicated by the brain wave feature data of the subject on the basis of the boundary information.

According to an eighth aspect of the present invention, the brain disease diagnosis assistance system further includes a display unit which outputs a result of the determination by the determination unit, wherein the determination unit determines a brain disease indicated by the brain wave feature data of the subject while calculating a probability that the brain wave feature data of the subject corresponds to the brain disease, and the display unit outputs the probability.

According to a ninth aspect of the present invention, a brain disease diagnosis assistance method includes acquiring, by a brain disease diagnosis assistance system, a plurality of learning data items, each including brain wave feature data including feature amounts of brain waves extracted from the brain waves and disease information attached to the brain wave feature data, the disease information indicating a state of a brain disease corresponding to the brain wave feature data, and calculating an evaluation model that performs brain disease determination through machine learning with the plurality of learning data items, and then acquiring brain wave feature data of a subject and performing determination of a brain disease indicated by the brain wave feature data of the subject on the basis of the evaluation model.

According to a tenth aspect of the present invention, a program allows a computer of a brain disease diagnosis assistance system to execute a function to acquire a plurality of learning data items, each including brain wave feature data including feature amounts of brain waves extracted from the brain waves and disease information attached to the brain wave feature data, the disease information indicating a state of a brain disease corresponding to the brain wave feature data, and to calculate an evaluation model that performs brain disease determination through machine learning with the plurality of learning data items, and a function to acquire brain wave feature data of a subject and to perform determination of a brain disease indicated by the brain wave feature data of the subject on the basis of the evaluation model.

According to an eleventh aspect of the present invention, a recording medium stores a program allowing a computer of a brain disease diagnosis assistance system to execute a function to acquire a plurality of learning data items, each including brain wave feature data including feature amounts of brain waves extracted from the brain waves and disease information attached to the brain wave feature data, the disease information indicating a state of a brain disease corresponding to the brain wave feature data, and to calculate an evaluation model that performs brain disease determination through machine learning with the plurality of learning data items, and a function to acquire brain wave feature data of a subject and to perform determination of a brain disease indicated by the brain wave feature data of the subject on the basis of the evaluation model.

[Advantageous Effects of Invention]

According to the brain disease diagnosis assistance system, the brain disease diagnosis assistance method, and the program described above, whether or not a subject is suffering from a brain disease can be determined simultaneously for a plurality of brain diseases on the basis of brain waves of the subject. In addition, an evaluation model used for the brain disease determination can be automatically created and therefore it is possible to save the time and effort of creating templates.

[Brief Description of Drawings]

Fig. 1 is a block diagram of a brain disease diagnosis assistance apparatus according to a first embodiment of the present invention.
Fig. 2 is a first diagram used to describe a process for calculating boundary information by the brain disease diagnosis assistance apparatus according to the first embodiment of the present invention.
Fig. 3 is a second diagram used to describe a process for calculating boundary information by the brain disease diagnosis assistance apparatus according to the first embodiment of the present invention.
Fig. 4 is a diagram used to describe an evaluation process performed by the brain disease diagnosis assistance apparatus according to the first embodiment of the present invention.
Fig. 5 is a flowchart of a process for evaluating brain wave feature data using the brain disease diagnosis assistance apparatus according to the first embodiment of the present invention.
Fig. 6 is a diagram illustrating an exemplary arrangement of electrodes for measuring brain waves in the first embodiment of the present invention.

Fig. 7 is a diagram illustrating exemplary feature amounts used in the brain disease diagnosis assistance apparatus according to the first embodiment of the present invention.

Fig. 8 is a first diagram used to describe an evaluation process performed by a brain disease diagnosis assistance apparatus according to a second embodiment of the present invention.

Fig. 9 is a second diagram used to describe the evaluation process performed by the brain disease diagnosis assistance apparatus according to the second embodiment of the present invention.

Fig. 10 is a flowchart of a process for evaluating brain wave feature data using the brain disease diagnosis assistance apparatus according to the second embodiment of the present invention.

Fig. 11 is a diagram illustrating a related-art dementia diagnosis method.

[Description of Embodiments]

<First Embodiment>

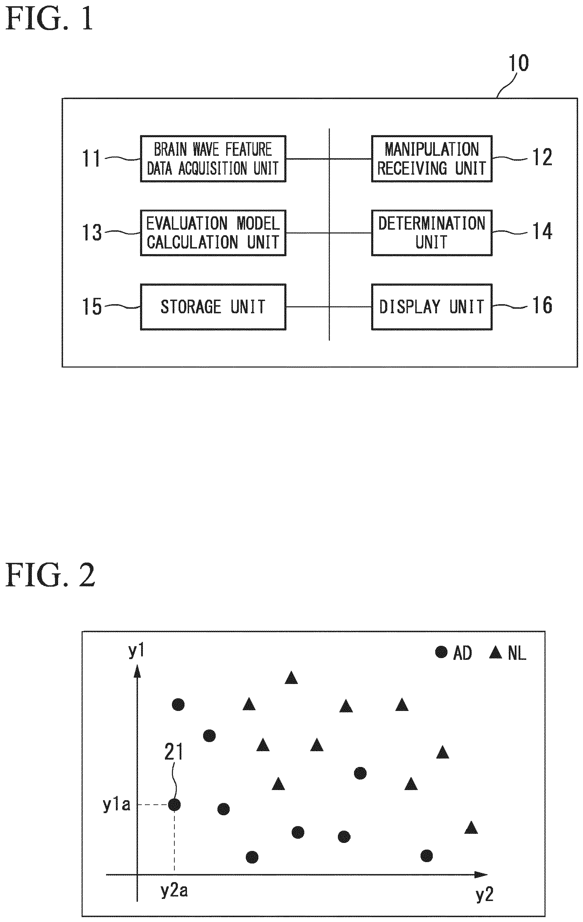

Hereinafter, a brain disease diagnosis assistance apparatus according to a first embodiment of the present invention is described with reference to Figs. 1 to 7.

Fig. 1 is a block diagram of the brain disease diagnosis assistance apparatus according to the first embodiment of the present invention.

The brain disease diagnosis assistance apparatus 10 of Fig. 1 is a system which presents a probability that a subject is suffering from dementia on the basis of brain waves of the subject. The brain disease diagnosis assistance apparatus 10 is, for example, a PC or a server device which can execute a program realizing this functionality of the system.

As shown in Fig. 1, the brain disease diagnosis assistance apparatus 10 includes a brain wave feature data acquisition unit 11, a manipulation-receiving unit 12, an evaluation model calculation unit 13, a determination unit 14, a storage unit 15, and a display unit 16.

The brain wave feature data acquisition unit 11 acquires brain wave feature data of the subject. The brain wave feature data is data including feature amounts of brain waves of the subject extracted from the brain waves of the subject. Brain waves are, for example, data recording the changes of an electrical potential difference between an electrode placed at an earlobe and another electrode from among electrodes placed on a head of the subject at specific positions according to the international 10-20 system. For example, according to the method of Patent Literature 1, the brain wave feature amounts include squares (sNAT) of electrical potentials measured at the positions where the electrodes are placed, in each of a plurality of frequency bands, and ratios of sNAT values between adjacent frequency bands (which relates to the smoothness of the brain waves). The brain wave feature data acquisition unit 11 acquires learning data including brain wave feature data and disease information attached thereto, the disease information indicating a brain disease that a person corresponding to the brain wave feature data has. Types of the brain disease include, for example, normal (without a brain disease), Alzheimer's dementia, vascular dementia, Lewy body dementia, depression, and schizophrenia.

The manipulation-receiving unit 12 receives an instructing manipulation that an operator has performed on the brain disease diagnosis assistance apparatus 10. Examples of the instructing manipulation include a manipulation for instructing that brain wave feature data be received and a manipulation for instructing that the brain wave feature data be evaluated.

The evaluation model calculation unit 13 calculates an evaluation model used for brain disease determination through machine learning with a plurality of learning data items acquired by the brain wave feature data acquisition unit 11. For example, in the present embodiment, the evaluation model calculation unit 13 calculates boundary information that separates the plurality of learning data items into portions corresponding respectively to the types of disease information attached to the plurality of learning data items. Machine learning used by the evaluation model calculation unit 13 is, for example, that of a support vector machine (SVM). Through the SVM, it is possible to calculate boundary information of multi-dimensional feature amounts. The boundary information is, for example, a function representing a separation boundary surface which separates brain wave feature data of normal persons and brain wave feature data of patients with Alzheimer's dementia.

The determination unit 14 acquires the brain wave feature data of the subject and performs determination of a brain disease indicated by the brain wave feature data of the subject on the basis of the evaluation model calculated by the evaluation model calculation unit 13. For example, in the present embodiment, the determination unit 14 evaluates brain wave feature data of a subject on the basis of the boundary information that the evaluation model calculation unit 13 has calculated based on the learning data. The evaluation includes, for example, determining a brain disease corresponding to brain wave feature data of a subject from among a plurality of brain diseases and calculating a probability that the brain wave feature data of the subject indicates the determined brain disease.

The storage unit 15 stores a variety of information such as brain wave feature data of a plurality of subjects and boundary information calculated by the evaluation model calculation unit 13.

The display unit 16 outputs an evaluation result of the determination unit 14 to a display device connected to the brain disease diagnosis assistance apparatus 10.

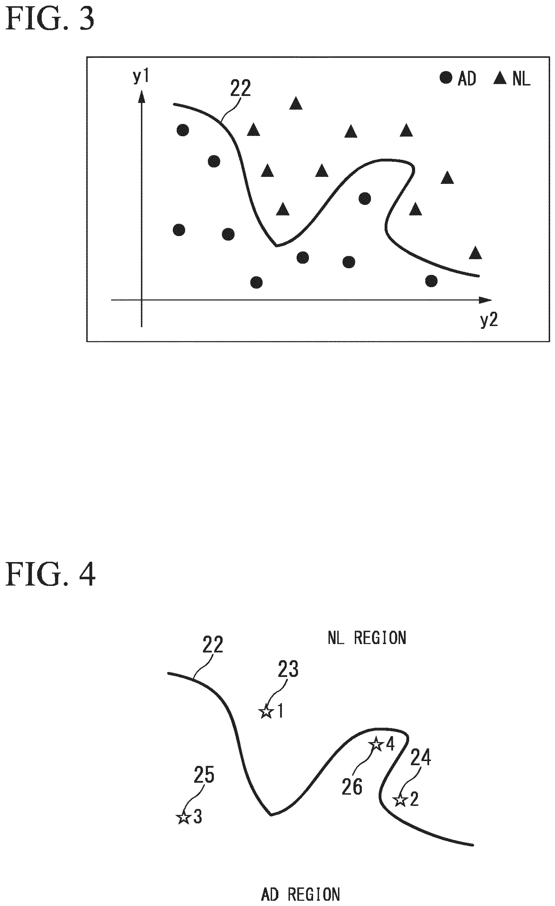

A process for calculating boundary information according to the first embodiment of the present invention will now be described with reference to Figs. 2 and 3.

Fig. 2 is a first diagram used to describe a process for calculating boundary information by the brain disease diagnosis assistance apparatus according to the first embodiment of the present invention.

First, x "learning data" items, each including a set of "brain wave feature data (= BF)" and "disease information (Label)," are prepared, where the brain wave feature data (BF) is a set of y brain wave feature amounts of a subject (such as the intensities of each frequencies of brain waves at the positions of electrodes for measurement) and the disease information (Label) represents a disease state of the subject. For example, AD is attached to the disease information when the subject has Alzheimer's dementia and NL is attached to the disease information when the subject is a normal person. In the present embodiment, it is assumed that the normal person is a person who is not suffering from Alzheimer's dementia. Such learning data of x subjects (LDx) is represented as follows.

$$LD1 = BF \{p11, p12, p13 \ldots p1y\} \text{ Label "AD"}$$

$$LD2 = BF \{p21, p22, p23 \ldots p2y\} \text{ Label "NL"}$$

$$\ldots$$

$$LDx = BF \{px1, px2, px3 \ldots pxy\} \text{ Label "AD"}$$

For ease of explanation, an example in which y=2 is described here. The diagram of Fig. 2 shows that learning data (y=2) of a plurality of patients with Alzheimer's dementia and normal persons is mapped in a two-dimensional space. In Fig. 2, each of circular points and triangular points represents learning data of a person. Each of the circular points is learning data of a patient with Alzheimer's dementia (AD). Each of the triangular points is learning data of a normal person (NL). The vertical-axis value represents a first brain wave feature amount that constitutes the brain wave feature data and the horizontal-axis value represents a second brain wave feature amount that constitutes the brain wave feature data. For example, a circular point 21 represents that a first brain wave feature amount of this person is "y1a," a second feature amount is "y2a," and the disease information is "AD."

First, the evaluation model calculation unit 13 receives learning data of a plurality of subjects that has been prepared in advance.

Fig. 3 is a second diagram used to describe a process for calculating boundary information by the brain disease diagnosis assistance apparatus according to the first embodiment of the present invention.

Upon receiving a plurality of learning data items, the evaluation model calculation unit 13 performs calculation of machine learning on brain wave feature data (BF) included in each of the plurality of learning data items, assuming the brain wave feature data (BF) as "y-dimensional data" (y=2), to calculate boundary information of "AD-like values" and "NL-like values." This can be realized, for example, by a machine learning logic which is called a support vector machine (SVM). The boundary information is calculated, for example, as a function representing a separation boundary surface. The evaluation model calculation unit 13 allows the calculated boundary information to be written to and stored in the storage unit 15. In the case of Fig. 3, the boundary information is represented, for example, by a boundary line 22.

Once the evaluation model calculation unit 13 has calculated boundary information from a plurality of learning data items and has then stored the same in the storage unit 15, the brain disease diagnosis assistance apparatus 10 can evaluate, for brain wave feature data (BFx) with no disease information attached thereto, whether the brain wave feature data BFx corresponds to AD or NL and a probability of the brain wave feature data BFx corresponding to AD or NL.

Fig. 4 is a diagram used to describe an evaluation process performed by the brain disease diagnosis assistance apparatus according to the first embodiment of the present invention.

When the evaluation model calculation unit 13 has calculated boundary information, the operator inputs brain wave feature data "BFx" of a subject to be diagnosed to the brain disease diagnosis assistance apparatus 10. The brain wave feature data acquisition unit 11 acquires and outputs the brain wave feature data "BFx" to the determination unit 14. The determination unit 14 reads the boundary information calculated by the evaluation model calculation unit 13 from the storage unit 15 and determines one of the portions separated by the calculated boundary to which the brain wave feature data "BFx" is classified. The determination unit 14 can calculate a probability that the brain wave feature data "BFx" of the subject to be diagnosed corresponds to AD or NL on the basis of how far the brain wave feature data "BFx" is from the boundary.

In Fig. 4, brain wave feature data $BF_1$ of subject 1 (shown as a star point 23) is present in the NL region, far from a boundary line 22, and therefore the determination unit 14 evaluates that there is a high probability of $BF_1$ corresponding to NL and the probability is, for example, 70%. Brain wave feature data $BF_2$ of subject 2 (shown as a star point 24) is present in the NL region, near the boundary line 22, and therefore the determination unit 14 evaluates that the probability of $BF_2$ corresponding to NL is high (for example, 60%) but there is a possibility that the identification is incorrect. Similarly, brain wave feature data $BF_3$ of subject 3 (shown as a star point 25) is present in the AD region, far from the boundary line 22, and therefore the determination unit 14 evaluates that there is a high probability of $BF_3$ corresponding to AD and the probability is, for example, 80%. Likewise, brain wave feature data $BF_4$ of subject 4 (shown as a star point 26) is present in the AD region, near the boundary line 22, and therefore the determination unit 14 evaluates that the probability of $BF_4$ corresponding to AD is high (for example, 55%) but there is a possibility that the identification is incorrect. A numerical value expressing a probability such as 70% can be calculated, for example, through an SVM technique.

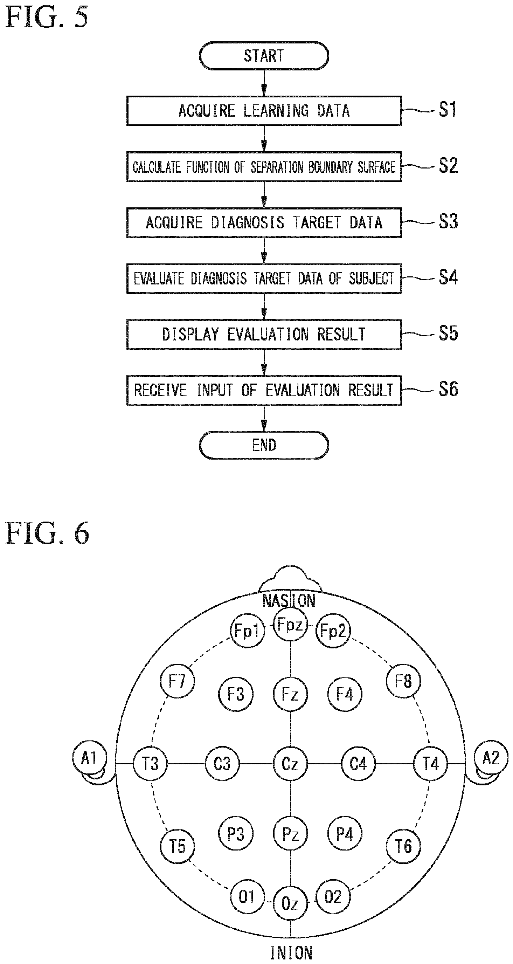

In the present embodiment, boundary information of AD and NL is obtained and whether brain wave feature data corresponds to AD or NL is evaluated using the boundary information as described above. Therefore, it is possible to avoid the problem that an "average AD" and an "average NL" are determined to flat the characteristics of AD or NL as in the related art. In addition, boundary information can be calculated through a machine learning technique and therefore there is no inconvenience of having to extract appropriate data through trial and error and accumulation of know-how. In addition, brain wave feature data of the present embodiment is measured at the positions of electrodes arranged according to the international 10-20 system of the international standard and 2 additional positions, i.e., measured at a total of 21 positions, and data acquired by dividing each of the values measured at the positions into respective values of a plurality of frequency characteristics is also used as the brain wave feature data. This increases the number of dimensions (y), for example, to 420 dimensions. However, using a machine learning technique such as that of an SVM can calculate the function of the boundary surface in multiple dimensions. An example arrangement of electrodes in the present embodiment will be described later with reference to Fig. 6.

Fig. 5 is a flowchart of a process for statistically evaluating brain wave feature data using the brain disease diagnosis assistance apparatus according to the first embodiment of the present invention.

A process for calculating boundary information and a process for evaluating dementia by the brain disease diagnosis assistance apparatus 10 are described below with reference to Fig. 5.

Here, it is assumed that an interface image that is to be manipulated by an operator is displayed on a display device connected to the brain disease diagnosis assistance apparatus 10, buttons such as "calculate boundary information," "evaluate diagnosis target data," "attach disease information of AD," and "attach disease information of NL" are displayed on the interface image, and the operator can instruct the brain disease diagnosis assistance apparatus 10 to perform corresponding processes by performing manipulations such as depressing the buttons with a mouse.

First, the operator inputs learning data of normal persons and patients with Alzheimer's dementia to the brain disease diagnosis assistance apparatus 10. For example, the learning data is given in an electronic file format and a single electronic file contains learning data of a plurality of normal persons and patients with Alzheimer's dementia. Here, it is assumed that disease information of "AD" or "NL" is attached to each learning data item. Feature data included in each learning data item is, for example, 420-dimensional data. The greater the amount of input learning data, the better. The brain wave feature data acquisition unit 11 acquires the learning data (step S1) and allows the acquired learning data to be written to and stored in the storage unit 15.

Then, when the operator depresses a button displayed as "calculate boundary information," the manipulation-receiving unit 12 receives this manipulation and instructs the evaluation model calculation unit 13 to calculate boundary information. The evaluation model calculation unit 13 reads the learning data written to the storage unit 15 and calculates a function of a separation boundary surface that divides the read learning data into AD and NL regions, for example, using an SVM technique (step S2). When the evaluation model calculation unit 13 has calculated a function representing the separation boundary surface, the evaluation model calculation unit 13 allows the calculated function (boundary information) to be written to and stored in the storage unit 15. The above is a description of the boundary information calculation process.

Then, a process for evaluating brain wave feature data of a new subject is performed. First, the operator inputs brain wave feature data of a subject to be diagnosed (i.e., diagnosis target data) to the brain disease diagnosis assistance apparatus 10. The diagnosis target data is given, for example, in an electronic file format. A single electronic file contains, for example, 420-dimensional brain wave feature data of a single subject. The brain wave feature data acquisition unit 11 acquires the diagnosis target data (step S3) and allows the acquired diagnosis target data to be written to and stored in the storage unit 15.

Then, when the operator depresses a button displayed as "evaluate diagnosis target data," the manipulation-receiving unit 12 receives this manipulation and instructs the determination unit 14 to evaluate the diagnosis target data. The determination unit 14 evaluates whether the diagnosis target data corresponds to AD or NL and a probability of the diagnosis target data corresponding to AD or NL using the boundary information written to the storage unit 15, for example, through an SVM technique (step S4). In SVM, the probability is a value (i.e., a certainty factor) based on the distance of the diagnosis target data from the separation boundary surface.

The determination unit 14 outputs a result of the evaluation to the display unit 16. The display unit 16 displays the evaluation result of the diagnosis target data of the subject on the display device (step S5). For example, information such as "there is a high probability of diagnosis target data being NL and the probability is 70%" is displayed as the evaluation result.

A diagnostician (i.e., a doctor) determines whether or not the subject has Alzheimer's dementia in a comprehensive manner using the evaluation result displayed on the display unit 16 and other results such as interview or test results. According to the brain disease diagnosis assistance apparatus 10, the probability of the subject having Alzheimer's dementia determined from the brain wave feature data can be presented to the doctor and therefore it is possible to assist doctors in diagnosing whether or not patients have Alzheimer's dementia. It takes some minutes for the determination unit 14 to provide an evaluation. Thus, if brain wave feature data of the subject has been prepared in advance, the doctor can quickly obtain evaluation results from the brain disease diagnosis assistance apparatus 10. In addition, brain wave measurement devices are cheaper than other medical devices used for dementia diagnosis such as MRIs, and acquisition of brain waves is safe in that it causes few problems for the subject's health even when brain waves are repeatedly acquired. Accordingly, the brain disease diagnosis assistance apparatus 10 of the present embodiment that assists in brain wave-based diagnosis of brain diseases such as dementia can be easily introduced even by relatively small medical institutions, and introducing a diagnosis assistance system including the brain disease diagnosis assistance apparatus 10 allows even medical institutions in a town to relatively easily diagnose Alzheimer's dementia which has become a social problem. Further, brain waves which are used to directly identify activity of cranial nerves allow abnormalities to be identified more quickly than with symptoms such as a patient's behavior which can be identified by others. Therefore, using the brain disease diagnosis assistance apparatus 10 of the present embodiment can determine the probability of having Alzheimer's dementia before symptoms occur and can also help in early diagnosis of and early measures against Alzheimer's dementia. Further, through interviews only, it is often not possible to determine whether symptoms, which have been seen by others as developing symptoms of dementia, are really symptoms of dementia or is a symptom other than those of dementia such as elderly depression. The brain disease diagnosis assistance apparatus 10 provides information that assists in diagnosis of Alzheimer's dementia of a patient who develops symptoms which are not easily recognized.

Then, brain wave feature data acquired through diagnosis by the doctor is received as learning data and a process for recalculating boundary information is performed. When the doctor has performed diagnosis with diagnosis target data, the operator inputs a result of the diagnosis to the brain disease diagnosis assistance apparatus 10. For example, the operator depresses the button "attach disease information of AD" when the diagnosis result is AD and depresses the button "attach disease information of NL" when the diagnosis result is NL.

Then, the brain wave feature data acquisition unit 11 receives this button depression manipulation (step S6) and associates the disease information (of AD or NL) indicated by the depressed button and the diagnosis target data with each other and writes the associated disease information and diagnosis target data to the storage unit 15. Accordingly, the disease information is attached to the diagnosis target data of the subject and the number of learning data items is increased by one as new learning data is added. Then, the operator depresses a button displayed as "generate boundary information." In response to this, the evaluation model calculation unit 13 adds the new learning data item to the learning data which has been written to the storage unit 15 in step S1 and performs the boundary information generation process of step S2 again. The evaluation model calculation unit 13 allows the newly calculated boundary information to be written to and stored in the storage unit 15. As the learning data of the subject is added to the given learning data, the number of learning data items is increased. Accordingly, it is possible to increase the accuracy and reliability of the boundary information calculated by the evaluation model calculation unit 13.

The display unit 16 may display a screen for selecting learning data acquired in step S1 to allow the operator to select learning data in accordance with the doctor's instruction or the like. The brain wave feature data may exhibit a different behavior with a small difference in positions at which electrodes are arranged or depending on mounting adjustment of electrodes on the head. For example, while it is desirable that evaluation according to the present embodiment be performed on the basis of brain wave feature data measured in the operator or doctor's facilities, evaluation using brain wave feature data collected throughout the country may fail to obtain desired results. In this case, reference learning data may be allowed to be selected such that the doctor can more effectively utilize the brain disease diagnosis assistance apparatus 10 in diagnosis of dementia by selecting only a learning data group which is helpful in diagnosis.

Patterns of learning data to be selected may be registered in the storage unit 15. For example, when the doctor has empirically found that some patterns exist for Alzheimer's dementia, groups of learning data respectively suitable for detecting patterns may be extracted and registered as different patterns and the different patterns may be switched and used as learning data. This makes it possible to evaluate diagnosis target data of a single subject from various viewpoints such that the doctor can more correctly diagnose Alzheimer's dementia.

In addition, arbitrary feature amounts may be selectable, for example, from 420-dimensional feature amounts of brain wave feature data to be used such that a boundary information calculation process or an evaluation process can be performed not only using all feature amounts included in the brain wave feature data but also using only some of the feature amounts as exemplified in the following Fig. 6. Further, patterns of brain wave feature data used for a series of processes may be registered and the patterns may then be switched to perform a boundary information calculation process or the like. For example, a combination of brain wave feature data based on brain waves measured at a plurality of specific positions may be defined as a first pattern and a combination of brain wave feature data based on brain waves measured at a plurality of positions different from those used for the first pattern may be defined as a second pattern and then boundary information calculation or the like may be performed using each of these two types of pattern. Accordingly, even when evaluation is performed using the same learning data group, it is possible to perform evaluation from various viewpoints and thus to assist the doctor in more detailed diagnosis. Furthermore, using a pattern acquired by excluding "feature elements which do not contribute to disease identification" from those for identification evaluation can prevent noise due to feature elements that do not directly contribute to disease identification and thus an effect of increasing identification accuracy can then be expected.

In addition, in step S2 of calculating the function of the separation boundary surface, effective feature amounts for separating AD and NL may be dynamically extracted using a technique such as principal component analysis or random forest and a function of the separation boundary surface may be calculated using only the extracted feature amounts.

Exemplary feature amounts that are considered to be effective for identification of Alzheimer's dementia are described below with reference to an example in which the same 420 feature amounts as those of Patent Literature 1 are used.

Fig. 6 is a diagram illustrating an exemplary arrangement of electrodes for measuring brain waves in the first embodiment of the present invention.

In Fig. 6, A1, A2, Fp1, Fp2, Fpz, F7, F3, Fz, F4, F8, T3, C3, Cz, C4, T4, T5, P3, Pz, P4, T6, O1, Oz, and 02 denote positions at which electrodes are arranged on a head of a subject. Actually, 21 brain waves of Fp1 to 02 are obtained since A1 and A2 correspond to reference electrodes. Brain waves of 4.68 to 20.28 Hz obtained from these electrodes are Fourier-transformed such that each is decomposed into eleven frequencies at intervals of 1.58 Hz. A normalized value of the electrical potential of each frequency is squared. This is referred to as an "sNAT value." A ratio of sNAT values between adjacent frequencies is obtained, which is referred to as a "vNAT value." Here, when 10 sNAT values other than those of the final frequency (20.28 Hz) are used, 10 sNAT values and 10 vNAT values are obtained for each electrode. A total of $21 \times 10 \times 10$ (= 420)-dimensional feature amounts (i.e., feature amounts of a total of $21 \times 10 \times 10$ (= 420) dimensions) can be obtained since the number of electrodes is 21. Here, normalization includes calculating difference values between the measured brain wave data and brain wave data of a subject who is, for example, in a stable state to prevent variations among individuals. sNAT and vNAT values are exemplary feature amounts that constitute brain wave feature data. The feature amounts may include other feature amounts based on brain wave data obtained through measurement at the positions of the electrodes.

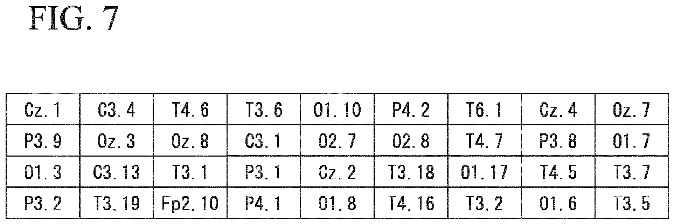

Fig. 7 is a diagram illustrating exemplary feature amounts used in the brain disease diagnosis assistance apparatus according to the first embodiment of the present invention.

Values shown in the table of Fig. 7 are feature amounts that have been determined to be effective for evaluation from among the above 420-dimensional feature amounts as a result of an evaluation method performed according to the present embodiment.

The feature amounts shown in the table of Fig. 7 are exemplary feature amounts effective for identification obtained through a random forest algorithm. For example, it is found that performing an Alzheimer's dementia evaluation process of the present embodiment using only 36-dimensional feature amounts shown in Fig. 7 provides desirable results compared to when all 420-dimensional feature amounts are used.

A symbol prior to a period in each value shown in Fig. 7 represents an electrode position in Fig. 6 and a numerical value subsequent to a period represents an sNAT or vNAT value. Specifically, 1 to 9 represent sNAT values in order of increasing frequency from the lowest frequency and 10 to 19 represent vNAT values in order of increasing frequency from the lowest frequency. For example, "C3.4" represents a 4th sNAT value from the lowest frequency at the "C3" electrode and "O1. 17" represents a 7th vNAT value from the lowest frequency at the "O1" electrode.

While the above description has been given with reference to assistance in diagnosis of Alzheimer's dementia as an example, the method of the present embodiment may also be applied to other dementia and brain disease symptoms such as depression or schizophrenia. That is, learning data including disease information indicating a certain brain disease attached thereto and learning data of persons who are suffering from the brain disease may be prepared in advance and the evaluation model calculation unit 13 may calculate boundary information separating the brain disease and others and the determination unit 14 may calculate whether or not brain wave feature data of a subject corresponds to the brain disease and a probability that brain wave feature data of the subject corresponds to the brain disease.

<Second Embodiment>

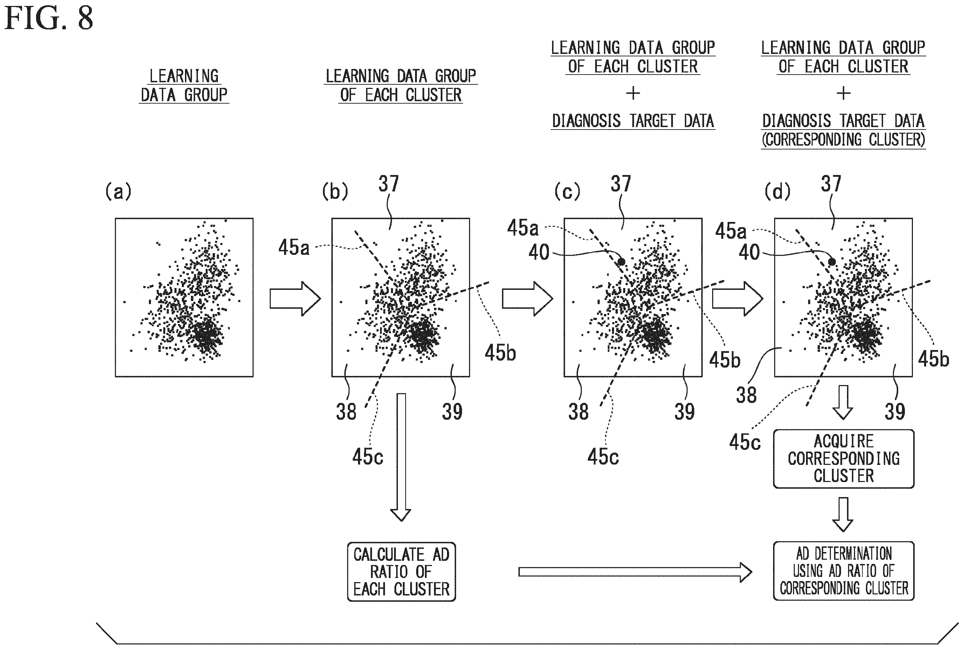

A brain disease diagnosis assistance apparatus according to a second embodiment of the present invention will now be described with reference to Figs. 8 to 10.

The first embodiment provides an evaluation method in which the presence or absence of Alzheimer's dementia is evaluated using learning data to which disease information of Alzheimer's dementia or others is attached. On the other hand, the second embodiment performs a process for classifying brain wave feature data of a subject as a similar group. This can improve the accuracy of evaluation in the case where feature data of brain waves including a plurality of dementias and a plurality of symptoms similar to dementia is included together in learning data.

An evaluation model calculation unit 13a of the present embodiment has a function to perform clustering to generate clusters as groups into which various brain wave feature data is classified, each of the groups including similar brain wave feature data. A determination unit 14a performs a process for determining a cluster, to which brain wave feature data of a subject belongs, from among the clusters generated by the evaluation model calculation unit 13a.

In addition, the evaluation model calculation unit 13a calculates a proportion of brain wave feature data, to which disease information of AD is attached, in a cluster which the brain wave feature data of the subject has been determined to be classified as by the determination unit 14a. The configuration is otherwise the same as that of the first embodiment.

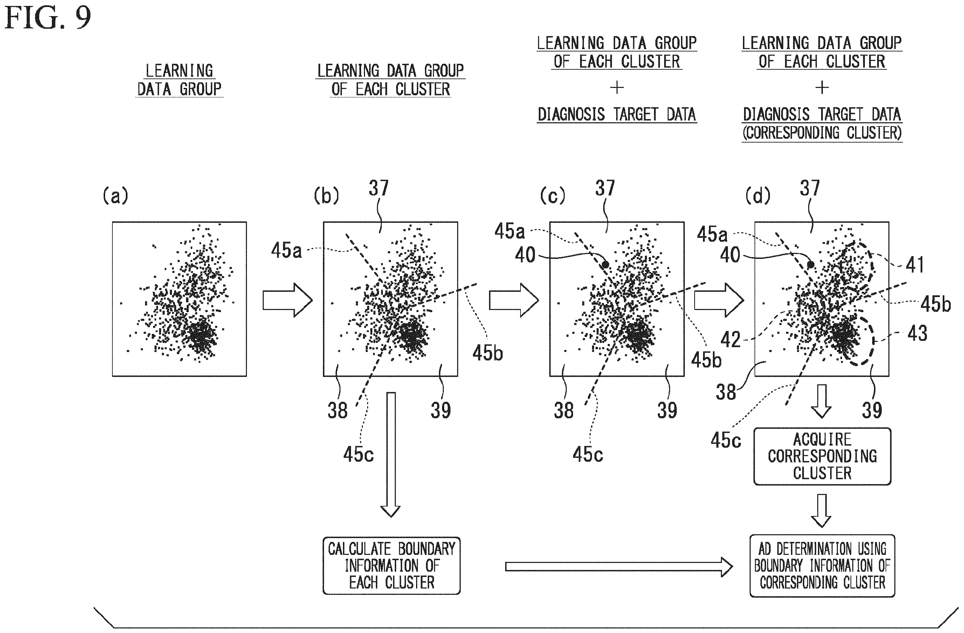

An evaluation process according to the second embodiment of the present invention is described below with reference to Figs. 8 and 9.

Fig. 8 is a first diagram used to describe an evaluation process performed by a brain disease diagnosis assistance apparatus according to the second embodiment of the present invention.

A diagram of Fig. 8a shows that learning data written to the storage unit 15 is mapped in two dimensions, similar to Figs. 2 and 3. Disease information indicating AD, NL, or other dementia may be attached to the learning data. The evaluation model calculation unit 13a classifies a plurality of learning data items into groups (clusters), each including similar learning data, through machine learning (clustering). For example, spectral clustering may be used as a clustering method.

Fig. 8b shows a result of clustering of learning data by the evaluation model calculation unit 13a. As shown, the learning data is classified into a cluster 37, a cluster 38, and a cluster 39 in this case of Fig. 8b. A dashed line 45a is a boundary line that separates learning data classified as the cluster 37 and learning data classified as the cluster 38. A dashed line 45b is a boundary line that separates learning data classified as the cluster 37 and learning data classified as the cluster 39. A dashed line 45c is a boundary line that separates learning data classified as the cluster 38 and learning data classified as the cluster 39. Here, the evaluation model calculation unit 13a calculates, for each type of disease information, a proportion of learning data, to which the type of disease information is attached, in each cluster with reference to disease information attached to learning data included in each cluster. For example, when 10 learning data items are classified as the cluster 37 and disease information of AD is attached to 4 of the 10 learning data items, disease information of NL is attached to 3 learning data items, disease information of Lewy body dementia is attached to 2 learning data items, and disease information of vascular dementia is attached to one learning data item, the evaluation model calculation unit 13a calculates a proportion of AD in the cluster 37 (i.e., an AD ratio) as 40%, a proportion of NL (i.e., an NL ratio) as 30%, a proportion of Lewy body dementia (i.e., a Lewy body dementia ratio) as 20%, and a proportion of vascular dementia (i.e., a vascular dementia ratio) as 10%.

Fig. 8c shows a state in which an operator has input brain wave feature data 40 of a subject to be diagnosed to the brain disease diagnosis assistance apparatus 10. When the brain wave feature data 40 of the subject has been input, the brain wave feature data acquisition unit 11 acquires and outputs the brain wave feature data of the subject to the determination unit 14a. The determination unit 14a determines a corresponding cluster to which the brain wave feature data 40 belongs from among the clusters 37 to 39, for example, using a clustering method such as a k-nearest neighbor technique.

Fig. 8d shows a result of the corresponding cluster determination by the determination unit 14a. Upon completing the determination, the determination unit 14 acquires the value of the AD ratio of the corresponding cluster calculated by the evaluation model calculation unit 13a and evaluates a probability that the brain wave feature data of the subject corresponds to AD on the basis of the acquired value. In this example, for example, the determination unit 14a evaluates that the probability of the brain wave feature data of the subject corresponding to AD is 40%.

According to this method, a probability of a subject having AD can be evaluated by classifying brain wave feature data of the subject according to the degree of similarity of the brain wave feature data, regardless of disease information attached to learning data. Accordingly, for example, when the AD ratio is evaluated as 40% in the above method even though it has been determined that the subject is NL in the evaluation method of the first embodiment, this can be referred to when considering the possibility that the subject will suffer from Alzheimer's dementia in the future. In addition, not only the possibility that AD may develop but also the possibility that other dementia may develop can be referred to by simultaneously displaying the proportion of learning data to which disease information of other dementia is attached.

Fig. 9 is a second diagram used to describe an evaluation process performed by the brain disease diagnosis assistance apparatus according to the second embodiment of the present invention.

Figs. 9a to 9d illustrate a method that combines the evaluation method using clustering described with reference to Fig. 8 and the evaluation method according to the first embodiment. When this method is used, it is assumed that the evaluation model calculation unit 13a has the function to calculate boundary information of the first embodiment in addition to and in conjunction with the function of the present embodiment. It is also assumed that the determination unit 14a has a function to evaluate brain wave feature data of a subject on the basis of the boundary information, similar to the first embodiment.

In the same manner as in the evaluation method described with reference to Fig. 8, learning data is clustered using a technique such as spectral clustering and a corresponding cluster to which brain wave feature data of a subject belongs is determined using a k-nearest neighbor technique or the like.

In the process of Fig. 9, when clustering of learning data has been completed, boundary information that separates AD and non-AD in each of the clusters 37-39 is calculated as shown in Fig. 9b, instead of obtaining an AD ratio or the like of each of the clusters 37-39. The evaluation model calculation unit 13a calculates the boundary information using both learning data to which disease information of AD is attached and other learning data according to a technique such as that of an SVM.

When it has been determined that the data of the subject belongs to, for example, the cluster 37, the determination unit 14a reads boundary information of the cluster 37 calculated by the evaluation model calculation unit 13a from the storage unit 15 and determines whether or not the brain wave feature data of the subject corresponds to AD according to a technique such as that of an SVM as shown in Fig. 9d. While the display unit 16 displays an AD ratio or the like as a result of evaluation of the brain wave data of the subject in Fig. 8, the display unit 16 displays a probability (a certainty factor) that the brain wave feature data of the subject calculated according to an SVM corresponds to AD in the case of Fig. 9.

According to this method, for example, even when learning data, for example, with an AD ratio of 50% is present in any of the clusters 37, 38, and 39, there is a possibility that evaluation accuracy of brain wave feature data of a subject can be increased. For example, there is a possibility that 3 types of pattern of brain wave feature data characterized by AD are present in the learning data, for example, with an AD ratio of 50% which is present in any of the clusters 37, 38, and 39. This example is shown in Fig. 9d. For example, it is assumed that disease information of AD is attached to learning data present in regions surrounded by dashed lines 41 to 43 in Fig. 9d. The dashed lines 41 to 43 are respective boundary information of the clusters 37 to 39. Here, if boundary information is calculated for all learning data as in the first embodiment, the calculated boundary information is unsuitable and there is a possibility that identification accuracy is reduced. Suitable boundary information is that of a boundary which maximizes a margin in the SVM. When brain wave feature data is previously classified into clusters, each including similar brain wave feature data, and boundary information is calculated with the classified clusters as in the method of Fig. 9, there is a possibility that more suitable boundary information can be calculated and it is thus possible to expect improvement of evaluation accuracy.

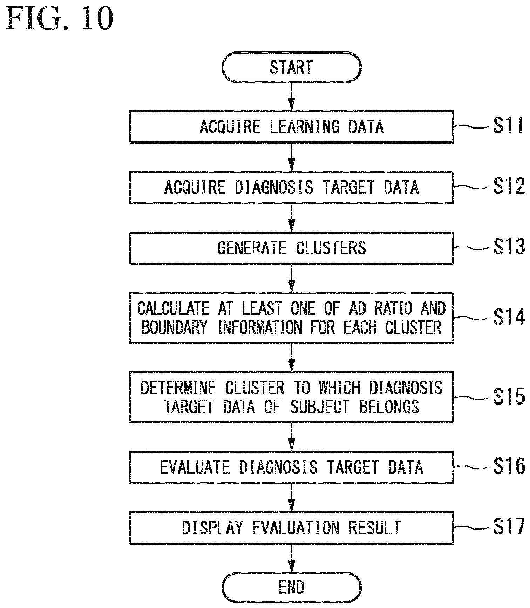

Fig. 10 is a flowchart of a process for evaluating brain wave feature data using the brain disease diagnosis assistance apparatus according to the second embodiment of the present invention.

First, the operator inputs learning data of normal persons and various patients to the brain disease diagnosis assistance apparatus 10. The learning data is given, for example, in an electronic file format. A single electronic file contains learning data to which a variety of disease information is attached. The variety of disease information is, for example, disease information of symptoms that are not those of dementia but are easily identified incorrectly as dementia, Alzheimer's dementia, Lewy body dementia, vascular dementia, and the like. The learning data of patients with Alzheimer's dementia may include learning data of severe patients, moderate patients, patients who are being treated with medication, patients who are not being treated with medication, or the like. The brain wave feature data acquisition unit 11 acquires learning data (step S11) and allows the acquired learning data to be written to and stored in the storage unit 15.

Then, the operator inputs diagnosis target data of a subject to be diagnosed to the brain disease diagnosis assistance apparatus 10. The brain wave feature data acquisition unit 11 acquires the diagnosis target data (step S12) and allows the acquired diagnosis target data to be written to and stored in the storage unit 15. Here, the brain wave feature data acquisition unit 11 records the diagnosis target data separately from the learning data acquired in step S 11.

Then, when the operator depresses a button displayed as "evaluate diagnosis target data," the manipulation-receiving unit 12 receives this manipulation and instructs the evaluation model calculation unit 13a to generate clusters (i.e., to perform clustering). The clusters are groups into which brain wave feature data of a plurality of subjects is classified based on the degree of similarity of feature amounts included in the brain wave feature data. The evaluation model calculation unit 13a reads the learning data received in step S11, which has been written to the storage unit 15, and generates a specific number of clusters from the read data according to a clustering method which is used in machine learning, such as, for example, spectral clustering (step S13). Here, it is assumed that the number of generated clusters has been set by the operator and then stored in advance in the storage unit 15. Learning data having similar brain wave feature data is classified as each cluster and a single cluster may include learning data corresponding to a variety of disease information of normal persons, patients with Alzheimer's dementia, and patients with Lewy body dementia.

Then, the evaluation model calculation unit 13a calculates at least one of an AD ratio and boundary information of each cluster (step S14). For example, in step S 13, the evaluation model calculation unit 13a calculates, for each cluster, a proportion of each type of disease information attached to learning data included in the cluster as described with reference to Fig. 8. The evaluation model calculation unit 13a allows the proportion of each type of disease information such as the calculated AD ratio in each cluster to be written to and stored in the storage unit 15. Alternatively, the evaluation model calculation unit 13a calculates boundary information that separates, for example, brain wave feature data to which disease information of a patient with Alzheimer's dementia is attached and brain wave feature data to which other disease information is attached as described with reference to Fig. 9. The boundary information is calculated, for example, using an SVM technique. Here, the boundary information may be calculated, for example, using data of all 420 dimensions or using feature amounts effective for AD identification from among feature amounts of 420 dimensions. Here, it is assumed that information indicating the effective feature amounts has been stored in the storage unit 15. Alternatively, feature amounts effective for evaluation according to a learning data group may be calculated each time as will be described later. One example of the feature amounts effective for AD identification is shown in Fig. 7. The evaluation model calculation unit 13a allows the calculated boundary information of each cluster to be written to and stored in the storage unit 15 in association with identification information of the cluster. The evaluation model calculation unit 13a may perform both the calculation of the proportion of each type of disease information and the calculation of boundary information. Then, the evaluation model calculation unit 13a instructs the determination unit 14a to evaluate the diagnosis target data.

The determination unit 14a then determines a cluster to which the diagnosis target data belongs using a technique such as a k-nearest neighbor technique (step S15).

The determination unit 14a then evaluates the diagnosis target data (step S16). In the case where the evaluation model calculation unit 13a has calculated the proportion of each type of disease information, the determination unit 14a evaluates a proportion of the diagnosis target data corresponding to AD. For example, in the case where the evaluation model calculation unit 13a has calculated AD ratios, the determination unit 14a reads, from the storage unit 15, an AD ratio corresponding to the cluster, to which the diagnosis target data has been determined to be classified, and evaluates that a probability of the diagnosis target data corresponding to AD is the AD ratio read from the storage unit 15. Alternatively, in the case where the evaluation model calculation unit 13a has calculated boundary information, the determination unit 14a reads, from the storage unit 15, boundary information corresponding to the cluster to which the diagnosis target data has been determined to be classified and evaluates whether the diagnosis target data corresponds to AD or NL and a probability (a certainty factor) of the diagnosis target data corresponding to AD or NL.

When evaluation has been done, the determination unit 14a outputs a result of the evaluation to the display unit 16. The display unit 16 displays the evaluation result of the diagnosis target data of the subject on the display device (step S17). For example, the display unit 16 displays positions of measurement of brain waves used as a basis for diagnosis, frequency bands, identification information of the cluster to which the diagnosis target data belongs, a probability that the diagnosis target data indicates AD, or the like. The probability that the diagnosis target data indicates AD is the AD ratio or the certainty factor described above. When performing the evaluation of step S16, the determination unit 14a may extract brain wave feature data included in learning data most similar to the brain wave feature data of the subject according to a technique such as pattern matching and the display unit 16 may display the brain wave feature data extracted by the determination unit 14a and disease information attached to the extracted data.

The doctor diagnoses a symptom of the subject on the basis of the information displayed by the display unit 16 and other test information. For example, in the case where brain wave feature data most similar to the brain wave feature data of the subject and disease information attached to the most similar brain wave feature data are displayed and the displayed disease information indicates Lewy body dementia, the doctor may diagnose referring to the fact that the brain wave feature data of the subject is similar to brain wave feature data of a patient with Lewy body dementia. In addition, when the proportion of dementia other than Alzheimer's dementia in the cluster to which the brain wave feature data of the subject is classified is displayed, the doctor may refer to this, for example, when considering the possibility that the subject may develop dementia other than Alzheimer's dementia.

The following procedure may be performed as a modified example of the process flow of Fig. 10. First, the AD ratio or the like is calculated in step S 14 and, when the AD ratio is, for example, 90%, in the evaluation of step S16, it is assumed that the brain wave feature data of the subject indicates AD and the display unit 16 outputs the evaluation result, terminating the process flow. When the AD ratio is, for example, 50%, it is determined that an additional evaluation process is required and boundary information that separates AD and non-AD is additionally calculated and the brain wave feature data of the subject is evaluated using an SVM or the like.

In step S 13, the number of generated clusters may be changed and processes subsequent to step S 13 may be repeatedly performed.

There is a plurality of types of dementia. Typical ones are Alzheimer's dementia, Lewy body dementia, and vascular dementia. These types of dementia are often indistinguishable simply by others looking. It is important to diagnose the correct type of dementia since different treatment methods are applied to different types of dementia. Thus, when one wishes to discriminate between a plurality of types of dementia using brain wave feature data, it is possible to consider, for example, manually selecting templates of the types of dementia and comparing the selected templates with brain wave feature data of a subject to determine the type of dementia. However, brain wave feature data corresponding to each type of dementia does not always have uniform features and thus it is difficult to manually create templates. According to the present embodiment, by performing clustering using brain wave feature data of patients with various types of dementia, it is possible to determine the type of dementia which the subject has and to calculate respective probabilities of the subject suffering from the plurality of types of dementia without conducting troublesome tasks. In addition, by preparing brain wave feature data of patients who have the same type of dementia but are in various states such as severe, moderate, or being under medication, it is possible to display, when a patient is suffering from dementia, information indicating whether the symptoms are moderate or severe or the like.

Also in the present embodiment, learning data used in the processes subsequent to step S 12 may be selected from the learning data acquired in step S 11.

All feature amounts or only some thereof may be used for the brain wave feature data used in the clustering of step S 13. In the step S 14 of generating clusters, for example, in the case where using feature amounts of all 420 dimensions is unsuccessful in clustering, feature amounts effective for clustering may be dynamically extracted using a technique such as principal component analysis or random forest and clusters may be generated using only the extracted feature amounts. In addition, in the case where boundary information is calculated using an SVM in step S16 of evaluating diagnosis target data, feature amounts effective for separation may be extracted using random forest or the like to calculate boundary information, similar to the first embodiment. This makes it possible to use effective feature amounts specific to each cluster, thereby increasing evaluation accuracy.

By changing such used learning data or feature amounts, it is possible to evaluate diagnosis target data from various viewpoints.

Although the present embodiment has been described with reference to diagnosis assistance for Alzheimer's dementia as an example, the present embodiment may also be applied to other dementia and brain disease symptoms such as depression and schizophrenia. In this case, probabilities of suffering from one or more of a plurality of brain diseases can be simultaneously calculated using learning data to which disease information of a variety of brain diseases is attached.

The flows of the processes of the brain disease diagnosis assistance apparatus 10 described above are stored in the form of a program in a computer-readable recording medium and the processes are performed by a computer of the brain disease diagnosis assistance apparatus 10 reading and executing the program. Here, the term "computer-readable recording medium" refers to a magnetic disk, a magneto-optical disk, a CD-ROM, a DVD-ROM, a semiconductor memory, or the like. The computer program may also be transmitted to a computer via communication lines and the computer may execute the program upon receiving the program.

The above program may be one for realizing a part of the functionality described above.

The above program may also be a so-called differential file (differential program) which is able to realize the functionality described above in combination with a program which has already been recorded in a computer system.

The brain disease diagnosis assistance apparatus 10 may be constructed using a single computer and may be constructed using a plurality of computers that are communicably connected.

The elements in the above embodiments may be appropriately replaced with well-known elements without departing from the nature of the present invention. The technical range of the present invention is not limited to the above embodiments and various changes may be made without departing from the nature of the present invention. For example, the evaluation model calculation unit 13 with a high computational load may be configured such that the evaluation model calculation unit 13 is divided by functionality into an evaluation model calculation unit 13b for SVM and an evaluation model calculation unit 13c for cluster generation and these two evaluation model calculation units are mounted on different PCs or the like. The brain disease diagnosis assistance apparatus 10 is an example of the brain disease diagnosis assistance system. The manipulation-receiving unit 12 is an example of the input unit.

[Industrial Applicability]

According to the brain disease diagnosis assistance system, the brain disease diagnosis assistance method, and the program described above, whether or not a subject is suffering from a brain disease can be determined simultaneously for a plurality of brain diseases on the basis of brain waves of the subject. In addition, an evaluation model used for the brain disease determination can be automatically created and therefore it is possible to save the time and effort of creating templates.

[Reference Signs List]

| | |
|---|---|
| 10 | Brain disease diagnosis assistance apparatus |
| 11 | Brain wave feature data acquisition unit |
| 12 | Manipulation-receiving unit |
| 13 | Evaluation model calculation unit |
| 14 | Determination unit |
| 15 | Storage unit |
| 16 | Display unit |

**Claims**

1.  A brain disease diagnosis assistance system, comprising:

    an evaluation model calculation unit which acquires a plurality of learning data items, each including brain wave feature data including feature amounts of brain waves extracted from the brain waves and disease information attached to the brain wave feature data, the disease information indicating a state of a brain disease corresponding to the brain wave feature data, and calculates an evaluation model that performs brain disease determination through machine learning with the plurality of learning data items; and
    a determination unit which acquires brain wave feature data of a subject and performs determination of a brain disease indicated by the brain wave feature data of the subject on the basis of the evaluation model.

2.  The brain disease diagnosis assistance system according to Claim 1, further comprising an input unit which receives an input of the disease information to be attached to the brain wave feature data of the subject,
    wherein the input unit allows a new learning data item including the brain wave feature data associated with the received disease information to be stored in a storage unit, and
    the evaluation model calculation unit adds the new learning data item to the plurality of learning data items to perform calculation of an evaluation model.

3.  The brain disease diagnosis assistance system according to Claim 1 or 2, wherein a plurality of the feature amounts are included in the brain wave feature data, and, for brain wave feature data included in each of the plurality of learning data items, the evaluation model calculation unit extracts one or a plurality of feature amounts effective for calculating an evaluation model and calculates the evaluation model using only the extracted feature amounts.

4.  The brain disease diagnosis assistance system according to any one of Claims 1 to 3, wherein information indicating one or a plurality of types of brain disease is included in disease information of the plurality of learning data items, the evaluation model calculation unit calculates boundary information that separates the plurality of learning data items into portions corresponding respectively to types of disease information attached to the learning data items, and
    the determination unit performs determination of a brain disease indicated by the brain wave feature data of the subject on the basis of the boundary information.

5.  The brain disease diagnosis assistance system according to any one of Claims 1 to 3, wherein the evaluation model calculation unit classifies the plurality of brain wave feature data into a plurality of groups by clustering based on the feature amounts, and
    the determination unit determines a group to which the brain wave feature data of the subject is classified from among the groups into which the evaluation model calculation unit has classified the plurality of brain wave feature data.

6.  The brain disease diagnosis assistance system according to Claim 5, wherein information indicating one or a plurality of brain diseases is included in disease information of the plurality of learning data items,
    the evaluation model calculation unit calculates, for each type of disease information, a proportion of learning data to which the type of the disease information is attached in learning data included in the group to which the brain wave feature data of the subject has been determined to be classified by the determination unit, and
    the determination unit performs determination of a brain disease indicated by the brain wave feature data of the subject on the basis of the proportion.

7. The brain disease diagnosis assistance system according to Claim 5 or 6, wherein the evaluation model calculation unit calculates boundary information that separates the learning data included in the group to which the brain wave feature data of the subject has been determined to be classified by the determination unit into portions corresponding respectively to types of disease information attached to the learning data items, and
the determination unit performs determination of a brain disease indicated by the brain wave feature data of the subject on the basis of the boundary information.

8. The brain disease diagnosis assistance system according to any one of Claims 1 to 7, further comprising a display unit which outputs a result of the determination by the determination unit,
wherein the determination unit determines a brain disease indicated by the brain wave feature data of the subject while calculating a probability that the brain wave feature data of the subject corresponds to the brain disease, and
the display unit outputs the probability.

9. A brain disease diagnosis assistance method, comprising:

acquiring, by a brain disease diagnosis assistance system, a plurality of learning data items, each including brain wave feature data including feature amounts of brain waves extracted from the brain waves and disease information attached to the brain wave feature data, the disease information indicating a state of a brain disease corresponding to the brain wave feature data, and calculating an evaluation model that performs brain disease determination through machine learning with the plurality of learning data items, and
acquiring brain wave feature data of a subject and performing determination of a brain disease indicated by the brain wave feature data of the subject on the basis of the evaluation model.

10. A program allowing a computer of a brain disease diagnosis assistance system to execute:

a function to acquire a plurality of learning data items, each including brain wave feature data including feature amounts of brain waves extracted from the brain waves and disease information attached to the brain wave feature data, the disease information indicating a state of a brain disease corresponding to the brain wave feature data, and to calculate an evaluation model that performs brain disease determination through machine learning with the plurality of learning data items, and
a function to acquire brain wave feature data of a subject and to perform determination of a brain disease indicated by the brain wave feature data of the subject on the basis of the evaluation model.

11. A recording medium storing a program allowing a computer of a brain disease diagnosis assistance system to execute:

a function to acquire a plurality of learning data items, each including brain wave feature data including feature amounts of brain waves extracted from the brain waves and disease information attached to the brain wave feature data, the disease information indicating a state of a brain disease corresponding to the brain wave feature data, and to calculate an evaluation model that performs brain disease determination through machine learning with the plurality of learning data items, and
a function to acquire brain wave feature data of a subject and to perform determination of a brain disease indicated by the brain wave feature data of the subject on the basis of the evaluation model.

# FIG. 1

# FIG. 2

# FIG. 3

# FIG. 4

# FIG. 5

```
START
  │
  ▼
ACQUIRE LEARNING DATA ───── S1
  │
  ▼
CALCULATE FUNCTION OF SEPARATION BOUNDARY SURFACE ───── S2
  │
  ▼
ACQUIRE DIAGNOSIS TARGET DATA ───── S3
  │
  ▼
EVALUATE DIAGNOSIS TARGET DATA OF SUBJECT ───── S4
  │
  ▼
DISPLAY EVALUATION RESULT ───── S5
  │
  ▼
RECEIVE INPUT OF EVALUATION RESULT ───── S6
  │
  ▼
END
```

# FIG. 6

FIG. 7

| Cz. 1 | C3. 4 | T4. 6 | T3. 6 | 01. 10 | P4. 2 | T6. 1 | Cz. 4 | 0z. 7 |
|---|---|---|---|---|---|---|---|---|
| P3. 9 | 0z. 3 | 0z. 8 | C3. 1 | 02. 7 | 02. 8 | T4. 7 | P3. 8 | 01. 7 |
| 01. 3 | C3. 13 | T3. 1 | P3. 1 | Cz. 2 | T3. 18 | 01. 17 | T4. 5 | T3. 7 |
| P3. 2 | T3. 19 | Fp2. 10 | P4. 1 | 01. 8 | T4. 16 | T3. 2 | 01. 6 | T3. 5 |

FIG. 8

LEARNING
DATA GROUP

LEARNING DATA GROUP
OF EACH CLUSTER

LEARNING DATA GROUP
OF EACH CLUSTER
+
DIAGNOSIS TARGET DATA

LEARNING DATA GROUP
OF EACH CLUSTER
+
DIAGNOSIS TARGET DATA
(CORRESPONDING CLUSTER)

(a)

(b) 37

(c) 37

(d) 37

45a

45a
40

45a
40

45b

45b

45b

38          39

38          39

38          39

45c

45c

45c

ACQUIRE
CORRESPONDING
CLUSTER

CALCULATE AD
RATIO OF
EACH CLUSTER

AD DETERMINATION
USING AD RATIO OF
CORRESPONDING CLUSTER

EP 3 216 392 A1

# FIG. 9

LEARNING DATA GROUP | LEARNING DATA GROUP OF EACH CLUSTER | LEARNING DATA GROUP OF EACH CLUSTER + DIAGNOSIS TARGET DATA | LEARNING DATA GROUP OF EACH CLUSTER + DIAGNOSIS TARGET DATA (CORRESPONDING CLUSTER)

(a)    (b)    (c)    (d)

CALCULATE BOUNDARY INFORMATION OF EACH CLUSTER

ACQUIRE CORRESPONDING CLUSTER

AD DETERMINATION USING BOUNDARY INFORMATION OF CORRESPONDING CLUSTER

EP 3 216 392 A1

FIG. 10

```
              ┌─────────────┐
              │    START    │
              └─────────────┘
                     │
                     ▼
     ┌───────────────────────────────────┐
     │       ACQUIRE LEARNING DATA        │──── S11
     └───────────────────────────────────┘
                     │
                     ▼
     ┌───────────────────────────────────┐
     │    ACQUIRE DIAGNOSIS TARGET DATA   │──── S12
     └───────────────────────────────────┘
                     │
                     ▼
     ┌───────────────────────────────────┐
     │         GENERATE CLUSTERS          │──── S13
     └───────────────────────────────────┘
                     │
                     ▼
     ┌───────────────────────────────────┐
     │ CALCULATE AT LEAST ONE OF AD RATIO AND │──── S14
     │ BOUNDARY INFORMATION FOR EACH CLUSTER  │
     └───────────────────────────────────┘
                     │
                     ▼
     ┌───────────────────────────────────┐
     │ DETERMINE CLUSTER TO WHICH DIAGNOSIS │──── S15
     │  TARGET DATA OF SUBJECT BELONGS    │
     └───────────────────────────────────┘
                     │
                     ▼
     ┌───────────────────────────────────┐
     │     EVALUATE DIAGNOSIS TARGET DATA │──── S16
     └───────────────────────────────────┘
                     │
                     ▼
     ┌───────────────────────────────────┐
     │       DISPLAY EVALUATION RESULT    │──── S17
     └───────────────────────────────────┘
                     │
                     ▼
              ┌─────────────┐
              │     END     │
              └─────────────┘
```

FIG. 11

## INTERNATIONAL SEARCH REPORT

| | International application No. |
|---|---|
| | PCT/JP2015/082844 |

### A. CLASSIFICATION OF SUBJECT MATTER
*A61B5/0476*(2006.01)i, *A61B10/00*(2006.01)i

According to International Patent Classification (IPC) or to both national classification and IPC

### B. FIELDS SEARCHED

Minimum documentation searched (classification system followed by classification symbols)
A61B5/04-5/053, A61B10/00

Documentation searched other than minimum documentation to the extent that such documents are included in the fields searched

| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 2009-528103 A (Mentis Cura ehf.),<br>06 August 2009 (06.08.2009),<br>paragraphs [0001] to [0093]; fig. 1 to 24<br>& US 2009/0054740 A1<br>paragraphs [0001] to [0170]; fig. 1 to 24<br>& WO 2007/098957 A1   & EP 1830292 A1<br>& CA 2644601 A1   & CN 101421735 A | 1,3-4,9-11<br>2,5-6,8 |
| X<br>Y | JP 2001-309898 A (Takumi IKUTA),<br>06 November 2001 (06.11.2001),<br>paragraphs [0001] to [0086]; fig. 1 to 8<br>(Family: none) | 1,3-5,7,9-11<br>2,6,8 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 February 2016 (15.02.16) | 23 February 2016 (23.02.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

International application No.

PCT/JP2015/082844

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2007-125362 A (New York University),<br>24 May 2007 (24.05.2007),<br>paragraphs [0001] to [0066]; fig. 1 to 4<br>& US 2007/0100251 A1<br>paragraphs [0001] to [0068]; fig. 1 to 4<br>& WO 2007/053263 A2 | 1,3-5,7,9-11<br>2,6,8 |
| Y | JP 2009-066186 A (Sony Corp.),<br>02 April 2009 (02.04.2009),<br>paragraphs [0127] to [0128]<br>(Family: none) | 2,5-6,8 |
| Y | JP 2005-237781 A (Waseda University),<br>08 September 2005 (08.09.2005),<br>paragraphs [0032] to [0047]; fig. 6 to 8<br>& US 2005/0192502 A1<br>paragraphs [0047] to [0065]; fig. 6 to 8 | 5-6,8 |
| A | JP 2010-500052 A (Hypo-Safe A/S),<br>07 January 2010 (07.01.2010),<br>entire text; all drawings<br>& US 2009/0287107 A1 & WO 2007/144307 A2<br>& CN 101896115 A | 1-11 |
| A | JP 2005-198970 A (Konica Minolta Medical &<br>Graphic, Inc.),<br>28 July 2005 (28.07.2005),<br>paragraph [0075]<br>& US 2005/0169517 A1<br>paragraph [0112]<br>& EP 1557792 A2 | 4,7 |
| A | JP 2010-523226 A (New York University),<br>15 July 2010 (15.07.2010),<br>paragraphs [0020] to [0025]<br>& US 2008/0249430 A1<br>paragraphs [0023] to [0029]<br>& WO 2008/124566 A2 & CA 2682955 A1<br>& CN 101677775 A | 7 |

Form PCT/ISA/210 (continuation of second sheet) (January 2015)

**REFERENCES CITED IN THE DESCRIPTION**

**Patent documents cited in the description**

- JP 2014248953 A

- JP 4145344 B